# EUROPÄISCHE PATENTANMELDUNG

(11) **EP 2 433 564 A1**
(43) Veröffentlichungstag der Anmeldung: **28.03.2012**
(21) Anmeldenummer: 11179129.9
(22) Anmeldetag: 29.08.2011
(51) Int. Cl.: A61B 5/053, A61B 5/107, A61F 2/84

(54) **Positionierung von Kathetern mittels Impedanzmessung**

(30) Priorität: 23.09.2010 US 385567 P
(71) Anmelder: BIOTRONIK SE & Co. KG, 12359 Berlin (DE)
(72) Erfinder: Vollkron, Michael, 3021 Pressbaum (AT); Lippert, Michael, 91522 Ansbach (DE)
(74) Vertreter: Lindner-Vogt, Karin L.

(57) **Zusammenfassung**

Die Erfindung bezieht sich auf ein Verfahren zur Bestimmung der Lage eines Katheters in einem Blutgefäß relativ zu einer Veränderung des Gefäßquerschnitts, umfassend die Schritte: - Bereitstellung eines Katheters der mindestens zwei Elektroden aufweist, die während der Positionierung des Katheters im Blutgefäß mit dem umgebenden Blut elektrisch leitend kontaktierbar sind, wobei die beiden Elektroden auf dem Katheter entlang der longitudinalen Achse in einem festgelegten Abstand voneinander angebracht sind; - Vorschub des Katheters im zu behandelnden Gefäß auf die Veränderung des Gefäßquerschnitts zu; und - Messung der Impedanz über die beiden Elektroden während des Vorschubs des Katheters; sowie auf einen Katheter zur Verwendung in einem solchen Verfahren.

## Beschreibung

Die Angioplastie, auch perkutane transluminale Angioplastie (PTA) oder perkutane transluminale Coronarangioplastie (PTCA), ist ein Verfahren zur Erweiterung oder Wiedereröffnung von verengten oder verschlossenen Blutgefäßen (meistens Arterien, seltener auch Venen). Gebräuchliche Verfahren der Angioplastie sind die Ballondilatation und die Applikation von Stents.

Unter der Ballondilatation im Rahmen einer Angioplastie versteht man in der interventionellen Radiologie, der Kardiologie und der Angiologie eine Methode zur Aufdehnung krankhaft verengter Blutgefäße mittels eines Ballonkatheters, einem Gefäßkatheter mit daran angebrachtem Ballon, der sich erst an der verengten Stelle langsam unter hohem Druck (6―20 bar) entfaltet. Dadurch werden die Engstellen, die v. a. durch atherosklerotische Veränderungen (Gefäßverkalkung) entstehen, so gedehnt, dass sie den Blutstrom nicht mehr oder weniger stark behindern.

Dabei werden die Ballonkatheter fast immer von der Leiste aus über einen Führungsdraht und Führungskatheter in die Stenose (Engstelle) platziert und mit Druck aufgeblasen. Hierdurch wird meist die Engstelle beseitigt und eine Operation vermieden.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen rohrförmigen Grundkörper mit einer filigranen Tragstruktur aus metallischen Streben auf, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden.

Sowohl bei der Ballondilatation als auch bei der Applikation von Stents hängt der therapeutische Erfolg wesentlich von der korrekten Positionierung des Ballons oder des Stents relativ zur zu behandelnden Verengung des Blutgefäßes ab. Es ist also von wesentlicher Bedeutung, die Lage des Katheters während der Positionierung des Katheters und vor der therapeutischen Gefäßaufweitung, der eigentlichen Expansion des Stents oder Ballons, möglichst schnell und exakt zu bestimmen, so dass die therapeutische Gefäßaufweitung auch an der gewünschten Stelle im Gefäß erfolgt. Während für die Kontrolle der Expansion von Stents oder Ballons bereits Katheter und Verfahren vorgeschlagen wurden, die ohne eine zusätzliche Röntgenbelastung des Patienten auskommen, siehe z. B. in US 2010/0010612 A1, so erfolgt die Positionierung des Stents vor der Expansion im Wesentlichen durch bekannte bildgebende Verfahren, wie z. B. durch Röntgenuntersuchungen. Die bisherigen bildgebenden Verfahren haben einige Nachteile wie z. B. eine Verzögerung des Verfahrens, eine unzureichende Genauigkeit, zusätzliche Kosten, eine Belastung des Patienten beispielsweise mit einer Röntgendosis, um nur einige zu nennen.

Aufgabe der vorliegenden Erfindung ist es einen oder mehrere Nachteile des Standes der Technik zu mindern oder zu verhindern. Insbesondere sollen neue Wege bereitgestellt werden, einen Katheter in einem Blutgefäß zu positionieren.

Diese Aufgabe wird gelöst durch Bereitstellung eines Verfahrens zur Bestimmung der Lage eines Katheters in einem Blutgefäß relativ zu einer Veränderung des Gefäßquerschnitts, insbesondere einer Verengung oder Erweiterung des Blutgefäßes, umfassend die Schritte:
- Bereitstellung eines Katheters der mindestens zwei Elektroden aufweist, die während der Positionierung des Katheters im Blutgefäß mit dem umgebenden Blut elektrisch leitend kontaktierbar sind, wobei die beiden Elektroden auf dem Katheter entlang der longitudinalen Achse in einem festgelegten Abstand voneinander angebracht sind;
- Vorschub des Katheters im zu behandelnden Gefäß auf die Veränderung des Gefäßquerschnitts zu; und
- Messung der Impedanz über die beiden Elektroden während des Vorschubs des Katheters.

Das erfindungsgemäße Verfahren beruht auf der überraschenden Erkenntnis, dass sich die Abhängigkeit der Impedanz gemessen über zwei Elektroden eines Katheters vom Gefäßquerschnitt dazu nutzen lässt, die Lage des Katheters in einem Blutgefäß relativ zu einer Veränderung des Gefäßquerschnitts, insbesondere relativ zu einer Verengung oder Erweiterung des Blutgefäßes, zu bestimmen. Während des Vorschubs des Katheters im Blutgefäß ergibt sich eine Korrelation zwischen der gemessenen Impedanz über die zwei Elektroden des Katheters und dem Gefäßquerschnitt des gerade von dem mit den Elektroden besetzten Bereich des Katheters erreichten oder durchquerten Gefäßbereichs. Nimmt der Gefäßquerschnitt ab, so ist ein Anstieg in der Impedanz messbar, nimmt der Gefäßquerschnitt zu, so ist eine Abnahme der Impedanz messbar. Wird die gemessene Impedanz über dem Vorschub aufgetragen, so sind die Veränderungen in der Impedanz direkt nutzbar für die Bestimmung der Lage des Katheters relativ zu einer Veränderung des Gefäßquerschnitts. Dabei lässt sich aus dem gemessenen Impedanzverlauf über den Vorschub nicht nur die Lage des Katheters bestimmen, sondern es können auch Aussagen über die Lage von mit dem Katheter verbundenen Vorrichtungen wie z. B. darauf angebrachten oder gecrimpten Stents oder dilatierbaren Ballonbereichen getroffen werden. Somit eignet sich das erfindungsgemäße Verfahren auch insbesondere zur Positionierung von Stents oder dilatierbaren Ballons vor der Expansion. Es ist darüber hinaus auch möglich die Ausdehnung einer Veränderung des Gefäßquerschnitts, insbesondere einer Verengung oder einer Erweiterung, in einem Blutgefäß entlang der Längsachse des Blutgefäßes zu bestimmen.

Das erfindungsgemäße Verfahren erlaubt die Bestimmung der Lage eines Katheters in einem Blutgefäß relativ zu einer Veränderung im Gefäßquerschnitt, insbesondere einer Verengung oder Erweiterung des Blutgefäßes. Es wird also nicht unmittelbar die absolute räumliche Lage des Katheters bestimmt, sondern die relative Lage im Blutgefäß in Bezug auf eine Veränderung im Gefäßquerschnitt in diesem Blutgefäß. Das erfindungsgemäße Verfahren kann aber mit anderen Verfahren kombiniert werden und so auch zur Bestimmung der absoluten räumlichen Lage eines Katheters beitragen. Des Weiteren können verschiedene abgeleitete Größen mit Hilfe der Impedanzmessung erfasst werden, wie z.B: Herzfrequenz, Veränderungen des Gefäßzustandes entlang der Vorschubrichtung des Katheters, die Atemfrequenz wenn der Katheter sich im Pulmonalkreislauf befindet oder Pulslaufzeiten (mit mindestens drei Elektroden).

Unter einer Veränderung des Gefäßquerschnitts kann hier grundsätzlich jede Verengung und/oder Erweiterung des Querschnitts eines Blutgefäßes verstanden werden, unabhängig davon, ob es sich um eine pathologische Veränderung des Gefäßes handelt oder nicht. Insbesondere kann es sich bei der Veränderung des Gefäßquerschnitts um eine oder mehrere radialsymmetrische und/oder exzentrische Stenosen/Aneurysmen handeln.

Das erfindungsgemäße Verfahren kann ebenso zur Katheter-Positionierung relativ zu einem bereits liegenden elektrisch leitfähigen Stent angewandt werden (z. B. um einen ungenügend dilatierten Stent noch mal "nachzudilatieren"). Es ergeben sich andere Impedanzverläufe, die allerdings ebenfalls eindeutige Rückschlüsse auf die Position des Katheters relativ zum liegenden Stent zulassen.

Zunächst wird ein Katheter bereitgestellt, der mindestens zwei Elektroden aufweist. Grundsätzlich kann dabei jede Katheterart und/oder -technologie verwendet werden, vorausgesetzt der Katheter weist zwei Elektroden auf und ist derart ausgebildet, das über die beiden Elektroden eine Impedanz messbar und bestimmbar ist. Die beiden Elektroden sind auf dem Katheter dabei derart angebracht, dass diese während der Positionierung des Katheters im Blutgefäß elektrisch leitend mit dem umgebenden Blut in Kontakt stehen. Der Katheter und die Elektroden sind dabei derart ausgebildet, dass an die Elektroden, beispielsweise über besonders ausgestaltete Führungsmittel des Katheters, eine definierte Spannung angelegt werden kann und der Leitfähigkeitsverlauf über die Elektroden gemessen werden kann. Geeignete Katheter und Elektrodenanordnungen sind exemplarisch beispielsweise in US 2010/0010612 A1 beschrieben. Insbesondere können die Elektroden des Katheters elektrisch leitend mit einer Spannungsquelle und Strommesseinheit oder wahlweise mit einer Stromquelle und Spannungsmesseinheit verbindbar ausgestaltet sein. Die Elektroden können beispielsweise über leitfähige Beschichtungen an der Innenseite des Katheterlumens kontaktierbar sein. Sollte das Katheterlumen während des Vorschubs mit einer elektrisch leitenden Flüssigkeit gefüllt sein, so kann auch diese zur Kontaktierung der Elektroden verwendet werden. Alternativ oder zusätzlich können die Elektroden auch über den Führungsdraht des Katheters elektrisch kontaktierbar sein. Die elektrische Kontaktierung der beiden Elektroden kann aber auch über extra in der Katheterwand eingelassene oder integrierte Leitungen oder Beschichtungen hergestellt sein.

Die beiden Elektroden sind auf dem Katheter entlang der Längsachse des Katheters in einem festgelegten Abstand voneinander angeordnet, bevorzugt nahe des Endes des Katheters, das in das Blutgefäß eingeführt wird. Dabei kann der Abstand zwischen den beiden Elektroden so gewählt sein, dass zwischen den beiden Elektroden ein Stent angebracht oder gecrimpt werden kann und/oder der Katheter einen dilatierbaren Bereich, z. B. einen deflatierbaren Ballon, aufweist. Sollte der Katheter einen Stent zwischen den beiden Elektroden tragen, so kann der Stent derart auf dem Katheter befestigt sein, dass der Stent mit keiner Elektrode des Katheters elektrisch leitend verbunden ist, wobei der Stent selbst elektrisch leitfähig sein kann. Bevorzugt steht der Stent während des Vorschubs des Katheters im Blutgefäß mit dem umgebenden Blut derart in Kontakt, dass ein elektrisch leitender Kontakt zwischen Stent und umgebendem Blut ermöglicht ist.

Der Katheter kann aber auch mehr als zwei Elektroden zur Impedanzmessung aufweisen. Werden beispielsweise mehrere Elektroden auf unterschiedlichen lateralen Flächen des Katheters eingesetzt, z. B. vier Elektroden mit jeweils zwei Elektroden auf gegenüberliegenden Seiten des Katheters, so kann der Einfluss eines unerwünschten Gefäßwandkontaktes auf das gemessene Impedanzsignal verringert werden.

Im erfindungsgemäßen Verfahren wird der Katheter nach erfolgter Bereitstellung in das Gefäßsystem eingeführt und im zu behandelnden Blutgefäß auf die Veränderung des Gefäßquerschnitts zu, insbesondere auf die Verengung oder Erweiterung des Gefäßes zu, vorgeschoben. Dieser Vorschub kann über die üblichen Führungsmittel des Katheters erreicht werden. Grundsätzlich bestehen an die Führungsmittel des Katheters keine besonderen Anforderungen. Geeignete Führungsmittel sind dem Fachmann bekannt. Es ist aber von Vorteil, wenn der Katheter Mittel umfasst oder mit Mitteln kombinierbar ist, die eine möglichst genaue Bestimmung des Vorschubs erlauben. Bevorzugt können hierzu Mittel eingesetzt werden, die die Strecke des Vorschubs möglichst exakt messen. Insbesondere können diese Mittel derart ausgeführt sein, dass der Vorschub über diese Mittel direkt regel-und/oder steuerbar ist und ggf. automatisiert erfolgen kann.

Im erfindungsgemäßen Verfahren wird die Impedanz während des Vorschubs über die beiden Elektroden gemessen. Die Impedanzmessung kann kontinuierlich oder diskontinuierlich in vorher festgelegten Intervallen erfolgen. Dazu wird über die beiden Elektroden ein Messstrom eingespeist und der Spannungsabfall zwischen den beiden Elektroden gemessen und somit die Impedanz bestimmt. Vorrichtungen und Verfahren zur Messung der Impedanz bzw. bevorzugt zur Erfassung und Darstellung der Impedanz über dem Vorschub sind dem Fachmann bekannt und stellen lediglich bauliche Anpassungen und die geeignete Anordnung von bereits existierenden Vorrichtungen dar, die vom Fachmann im Rahmen von Routinetätigkeiten vorgenommen werden können. Zur Impedanzmessung werden bevorzugt möglichst geringe Messströme angelegt, besonders bevorzugt werden Messströme im Bereich von 1 µA bis 1 mA, insbesondere in Form von Pulsen mit Pulsbreiten im Bereich 10µs bis 1000µs oder wahlweise als Wechselstrom mit einer Frequenz im Bereich von 1kHz bis 100kHz verwendet. Dabei sollte die Kombination so gewählt sein, dass eine effektive Stimulation von Gewebe, insbesondere des Herzmuskels und von Nervenfasern, durch die Messpulse ausgeschlossen ist.

Im erfindungsgemäßen Verfahren kann zur Impedanzmessung ein Messgerät verwendet werden. Das Messgerät zur Impedanzmessung ist mit dem Katheter derart verbunden oder verbindbar, dass die Impedanz über die beiden Elektroden des Katheters messbar ist. Das Messgerät kann beispielsweise über Batterien, Akkumulatoren oder ein geeignetes Netzteil betrieben werden. Das Messgerät ist bevorzugt derart ausgeführt, dass es für mehrere Anwendungen wiederverwendet werden kann, es kann aber auch als Einmal- oder Wegwerfgerät ausgestaltet sein. Das Messergebnis kann visuell, z. B. als Diagramm, und/oder akustisch, z. B. als Ton oder variierte Tonhöhe, ausgebbar sein. Das Impedanzmessgerät kann zusätzlich mit weiteren Messeinrichtungen versehen oder kombinierbar sein, z. B. kann das Messgerät zusätzliche Vorrichtungen zur Positionsbestimmung, insbesondere zur bildgebenden Darstellung, des Katheters ausgerüstet sein. Das Impedanzmessgerät kann derart ausgeführt und konfiguriert sein, dass die Messdaten zur Lagebestimmung des Katheters automatisch ausgewertet werden. Diese Auswertung kann dem Arzt derart bereitgestellt und/oder vermittelt werden, dass der Arzt akustisch und/oder visuell auf Vorschläge zur optimalen Katheterposition hingewiesen wird und/oder auf einen zu kurzen oder zu langen Stent aufmerksam gemacht wird.

Wird das Impedanzmessgerät beispielsweise mit einer mechanischen regel- und/oder steuerbaren Vorschubeinrichtung kombiniert, vorzugsweise mit einer Vorschubeinrichtung mit Kraftrückmeldung, so kann eine vollautomatische Feinpositionierung des Katheters realisiert werden.

Das erfindungsgemäße Verfahren liefert einen Impedanzverlauf während des Vorschubs, der leicht interpretierbar ist. Bevorzugt kann der Impedanzverlauf als Impedanz aufgetragen über die Strecke des Vorschubs bestimmt und zur Lagebestimmung verwendet werden. Der gemessene Impedanzverlauf während des Vorschubs wird für die Bestimmung der Lage des Katheters im Blutgefäß entlang der Längsachse des Blutgefäßes und relativ zu einer Veränderung des Gefäßquerschnitts verwendet. Dabei gibt die Zunahme der Impedanz einen Hinweis darauf, dass eine der beiden Elektroden einen Bereich mit einem geringeren Gefäßquerschnitt erreicht hat, während eine Abnahme der Impedanz darauf schließen lässt, dass eine der Elektroden einen Bereich mit einem größeren Gefäßquerschnitt erreicht hat bzw. einen Bereich mit einem geringeren Gefäßquerschnitt verlassen hat. Da die gemessenen Impedanzverläufe sich aus einer Addition der Zustände an jeder der beiden Elektroden zusammensetzen, kann aus einem Impedanzverlauf unmittelbar bestimmt werden, ob sich keine, eine oder beide Elektroden im Bereich einer Gefäßverengung oder Gefäßerweiterung, also einem Bereich mit einem veränderten Gefäßquerschnitt, befinden. Da der Abstand der beiden Elektroden auf dem Katheter bekannt ist, kann das erfindungsgemäße Verfahren zur Bestimmung der Ausdehnung einer Blutgefäßverengung oder Blutgefäßerweiterung entlang der Längsachse des Blutgefäßes verwendet werden. Da sich mit dem erfindungsgemäßen Verfahren allgemein Bereiche in einem Blutgefäß mit veränderten Querschnitt identifizieren und charakterisieren lassen, kann das Verfahren auch zur Kontrolle des Therapieerfolges einer Stentapplikation oder Ballondilatation verwendet werden.

Das erfindungsgemäße Verfahren kann auch bei der Platzierung eines Stents in einem Blutgefäß eingesetzt werden, insbesondere zur Bestimmung der optimalen Lage vor der Expansion des Stents.

Der Katheter zur Verwendung im erfindungsgemäßen Verfahren kann zwischen den beiden Elektroden einen Stent tragen. Der Katheter kann dann mittels des erfindungsgemäßen Verfahrens derart im Blutgefäß positioniert werden, dass der Stent im Bereich der Verengung zu liegen kommt und anschließend dort durch Expansion appliziert wird.

In einer bevorzugten Ausführungsform wird ein Katheter bereitgestellt, der zwischen den beiden Elektroden einen leitfähigen Stent trägt. Dabei ist der elektrisch leitfähige Stent während der Positionierung des Katheters im Blutgefäß mit dem umgebenden Blut elektrisch leitend kontaktiert. Dies führt dazu, dass Eintritt und Austritt jeder der beiden Elektroden in bzw. aus dem Bereich der Veränderung des Gefäßquerschnitts jeweils zu einer stufenförmigen Veränderung der Impedanz über die beiden Elektroden führt. Insbesondere führt der Eintritt jeder der beiden Elektroden in den Bereich einer Verengung des Blutgefäßes unabhängig voneinander jeweils zu einer stufenförmigen Zunahme der Impedanz und der Austritt jeder der beiden Elektroden aus dem Bereich der Verengung des Blutgefäßes jeweils zu einer stufenförmigen Abnahme der Impedanz über die beiden Elektroden. Dies liegt daran, dass der Strom von den Elektroden nur einen kurzen Weg über das umgebende

Gewebe nehmen muss, um dann im Stent weiterfließen zu können. Deshalb fließen im Fall eines leitfähigen Stents in die gemessene Impedanz nur die elektrischen Eigenschaften des die beiden Übergangsbereiche zwischen Elektroden und Stent umgebenden Gewebes (Gefäßwand/Blut) ein. Anhand des Impedanzverlaufs lässt sich also eindeutig sagen, ob sich zu einem bestimmten Punkt des Vorschubs, also bei einer bestimmten Lage des Katheters entlang der Längsachse des Blutgefäßes, keine, eine oder beide Elektroden in einem Bereich mit einem geringeren Gefäßquerschnitt befinden. Anhand des Impedanzverlaufs über dem Vorschub ist also unmittelbar erkennbar, ob sich der zwischen den beiden Elektroden befindliche Stent im Bereich der zu behandelnden Verengung befindet oder nicht. Diese Erkenntnis lässt sich im erfindungsgemäßen Verfahren nutzen. Somit ist es möglich den Katheter im Blutgefäß relativ zu einer Veränderung des Gefäßquerschnitts derart zu positionieren, dass sich die Impedanz über die beiden Elektroden zwischen der stufenförmigen Impedanzveränderung der bereits aus dem Bereich der Veränderung des Gefäßquerschnitts austretenden ersten Elektrode und vor der stufenförmigen Impedanzveränderung durch die in den Bereich der Veränderung des Gefäßquerschnitts eintretenden zweiten Elektrode befindet. Insbesondere ist es möglich, den Katheter im Blutgefäß relativ zu einer Verengung derart zu positionieren, dass sich die Impedanz über die beiden Elektroden zwischen einer stufenförmigen Impedanzabnahme der bereits aus dem Bereich der Verengung austretenden ersten Elektrode und vor einer stufenförmigen Impedanzzunahme durch die in den Bereich der Verengung eintretende zweite Elektrode befindet. Ist dies der Fall, so befindet sich der Stent im Bereich einer Verengung des Gefäßquerschnitts. Wird der Katheter im Wesentlichen in der Mitte zwischen der stufenförmigen Impedanzabnahme der ersten Elektrode und der stufenförmigen Impedanzzunahme der zweiten Elektrode platziert, so befindet sich der Stent im Zentrum der Verengung und kann dort durch Expansion platziert werden.

Analog kann das beschriebene Verfahren verwendet werden, um eine lokale Erweiterung des Gefäßes zu lokalisieren. Die Impedanzverläufe sind dann entsprechend umgekehrt.

In einer anderen Ausführungsform des erfindungsgemäßen Verfahrens wird ein Katheter bereitgestellt, der keinen Stent trägt, oder einen im Wesentlichen nicht leitfähigen Stent trägt oder der Stent während der Positionierung des Katheters im Blutgefäß nicht mit dem umgebenden Blut elektrisch leitend in Kontakt steht. Dies führt dazu, dass Eintritt und Austritt jeder der beiden Elektroden in bzw. aus dem Bereich der Veränderung des Gefäßquerschnitts jeweils zu einer rampenförmigen Veränderung der Impedanz über die beiden Elektroden führt. Insbesondere kann der Eintritt jeder der beiden Elektroden in den Bereich einer Verengung des Blutgefäßes zu einer rampenförmigen Zunahme der Impedanz über die beiden Elektroden und der Austritt jeder der beiden Elektroden aus dem Bereich der Verengung des Blutgefäßes zu einer rampenförmigen Abnahme der Impedanz über die beiden Elektroden führen. Das liegt daran, dass in diesem Fall das umgebende Gewebe auch auf der ganzen Länge zwischen den beiden Elektroden zur gemessenen Impedanz beiträgt, wenngleich die direkte Nachbarschaft der Elektroden eine größere Gewichtung besitzt. Die schematisch "rampenförmig" genannten Impedanzverläufe müssen deshalb nicht linear verlaufen, zeigen aber in jedem Fall einen kontinuierlich ansteigenden bzw. abfallenden Verlauf und nicht nur eine Stufe. Anhand des Impedanzverlaufs lässt sich also wieder eindeutig sagen, ob sich zu einem bestimmten Punkt des Vorschubs, also bei einer bestimmten Lage des Katheters relativ zur Längsachse des Blutgefäßes, keine, eine oder beide Elektroden in einem Bereich mit einem geringeren Gefäßquerschnitt befinden. Anhand des Impedanzverlaufs über dem Vorschub ist also unmittelbar erkennbar, ob sich der zwischen den beiden Elektroden befindliche Stent im Bereich einer zu behandelnden Verengung befindet oder nicht. Diese Erkenntnis lässt sich im erfindungsgemäßen Verfahren nutzen. Somit ist es möglich, den Katheter im Blutgefäß derart zu positionieren, dass sich der Impedanzverlauf in einem Impedanzplateau befindet, welches sich zwischen einer rampenförmigen Impedanzzunahme und einer rampenförmigen Impedanzabnahme befindet. Ist dies der Fall, so befindet sich der Stent im Bereich der Verengung. Wird der Katheter im Wesentlichen im Impedanzverlauf in der Mitte des Plateaus platziert, so befindet sich der Stent im Zentrum der Verengung und kann dort durch Expansion in besonders vorteilhafter Weise platziert werden.

Analog kann auch dieses Verfahren verwendet werden, um eine lokale Erweiterung des Gefäßes zu lokalisieren. Die Impedanzverkäufe sind dann entsprechend umgekehrt.

Somit lässt sich das erfindungsgemäße Verfahren auch dazu verwenden, um zu bestimmen, ob ein Stent auch eine ausreichende Länge besitzt, um die zu behandelnde Verengung oder Erweiterung über die gesamte Ausdehnung in längsaxialer Richtung effektiv aufzuweiten oder zu stabilisieren.

Das erfindungsgemäße Verfahren kommt ohne die Anwendung von Röntgenstrahlung aus, so dass insgesamt bei der Applikation eines Stents der Patient einer geringeren oder keiner Röntgendosis ausgesetzt wird. Das erfindungsgemäße Verfahren begleitet den Vorschub des Katheters in Echtzeit und führt insgesamt nicht zu einer Verlängerung der Behandlungszeit.

Dadurch dass, das erfindungsgemäße Verfahren das Auffinden der optimalen Position für die Applikation des Stents oder des Ballons ermöglicht, bevor eine Expansion erfolgt, wird insgesamt die Zuverlässigkeit der Gefäßaufweitung und -stabilisierung durch Stents oder Ballonkatheter verbessert.

Durch die Möglichkeit der optimalen Positionierung, verbunden mit einer frühzeitigen Erkennung einer falschen Stentlänge führt der Einsatz eines erfindungsgemäßen Verfahrens insgesamt zu einem verbesserten Behandlungserfolg.

Das erfindungsgemäße Verfahren kann auch dazu verwendet werden Gefäßabzweigungen zu identifizieren, z. B. für das Identifizieren von geeigneten Implantationsorten, beispielsweise für die Implantation von Drucksensoren, Schrittmacher-, Defibrilator-, oder CRT-Elektroden.

Die vorliegende Erfindung bezieht sich auch auf einen Katheter zur Verwendung in einem erfindungsgemäßen Verfahren.

Figuren:

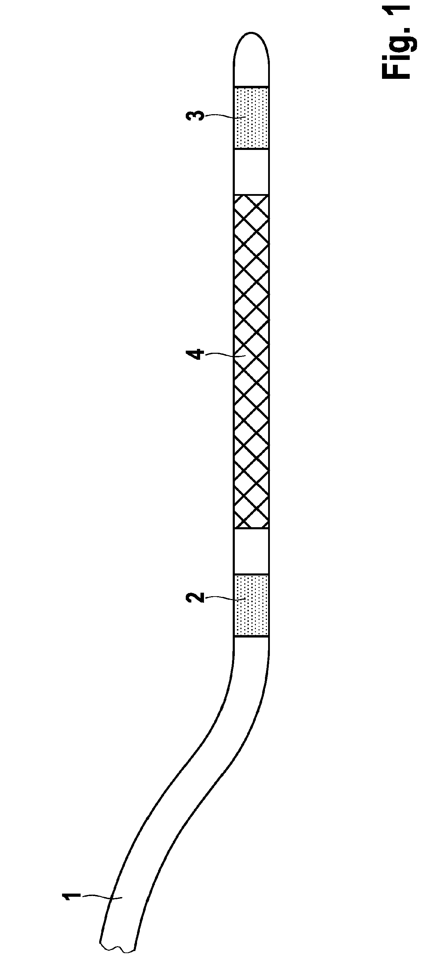

Fig. 1 zeigt eine schematische Darstellung eines Katheters zur Verwendung in einem erfindungsgemäßen Verfahren.

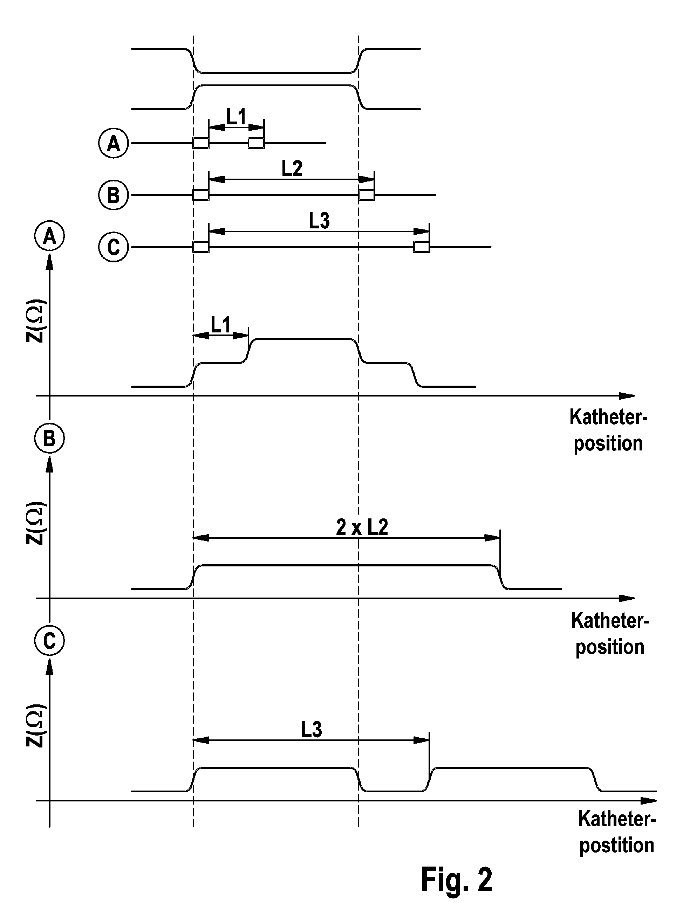

Fig. 2 zeigt einen Vergleich von Impedanzverläufen für unterschiedliche Abstände zwischen den beiden Elektroden gemessen für einen Katheter mit einem leitfähigen Stent. (A) Impedanzverlauf über dem Vorschub, wenn der Abstand zwischen den Elektroden des Katheters kleiner ist als der Bereich der Verengung; (B) Impedanzverlauf über dem Vorschub, wenn der Abstand zwischen den Elektroden des Katheters identisch ist mit dem Bereich der Verengung; (C) Impedanzverlauf über dem Vorschub, wenn der Abstand zwischen den Elektroden des Katheters größer ist als der Bereich der Verengung.

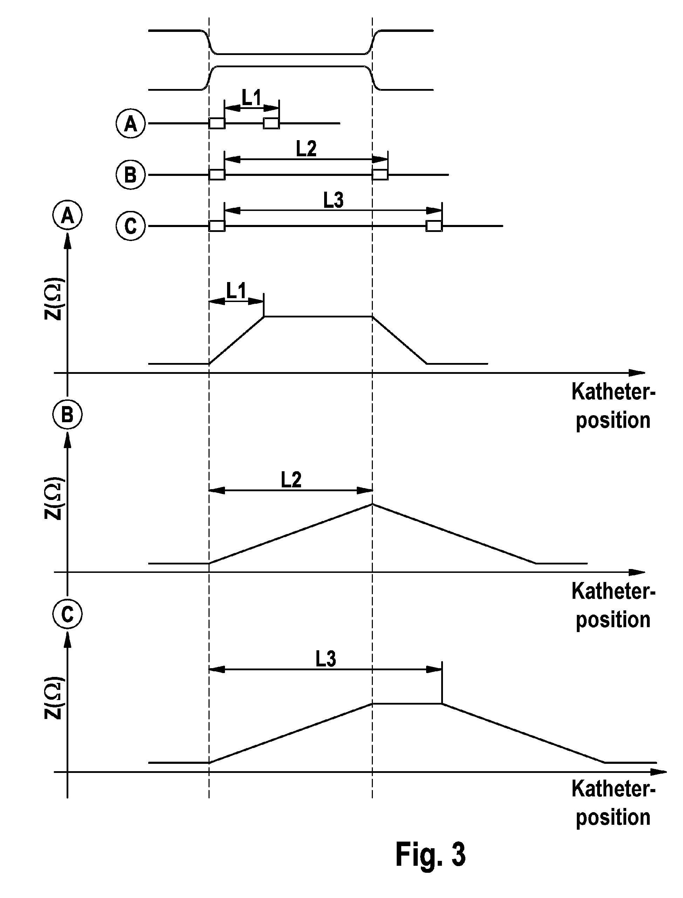

Fig. 3 zeigt einen Vergleich von Impedanzverläufen für unterschiedliche Abstände zwischen den beiden Elektroden gemessen für einen Katheter mit einem nicht-leitfähigen Stent oder nur mit einem Ballon. (A) Impedanzverlauf über dem Vorschub, wenn der Abstand zwischen den Elektroden des Katheters kleiner ist als der Bereich der Verengung; (B) Impedanzverlauf über dem Vorschub, wenn der Abstand zwischen den Elektroden des Katheters identisch ist mit dem Bereich der Verengung; (C) Impedanzverlauf über dem Vorschub, wenn der Abstand zwischen den Elektroden des Katheters größer ist als der Bereich der Verengung. Die hier schematisch als Geraden gezeichneten rampenförmigen Bereiche können in der Realität auch eine nichtlineare, aber dennoch kontinuierlich ansteigende bzw. abfallende Form annehmen.

Nachfolgend wird die Erfindung anhand von Ausführungsbeispielen näher erläutert.

### Ausführungsbeispiel

In Figur 1 ist ein Katheter schematisch dargestellt, der in einem erfindungsgemäßen Verfahren verwendet werden kann. Der Katheter umfasst einen Grundkörper 1 und weist nahe dem Ende, welches in das Blutgefäß eingeführt wird, zwei Elektroden 2 und 3 auf. Die beiden Elektroden 2 und 3 sind auf dem Grundkörper 1 in einem Abstand in längsaxialer Richtung angeordnet. Zwischen den beiden Elektroden 2 und 3 ist ein Stent 4 platziert, der nach erfolgter Positionierung des Stents durch Expansion im Blutgefäß applizierbar ist. Dieser Stent kann während der Positionierung des Katheters im Blutgefäß entweder elektrisch leitend mit dem umgebenden Blut in Kontakt stehen oder nicht.

Der Katheter aus Figur 1 kann nach einem erfindungsgemäßen Verfahren derart positioniert werden, dass sich der Stent 4 in einer optimalen Lage im Bereich einer Stenose befindet. Dazu wird der Katheter zunächst in das Gefäßsystem eingeführt und durch geeigneten Vorschub auf die zu behandelnde Verengung im Blutgefäß zu bewegt. Während des Vorschubs wird ein Messstrom eingespeist und kontinuierlich die Impedanz über die beiden Elektroden 2 und 3 gemessen. Anhand des Impedanzverlaufs über dem Vorschub ist es möglich die Lage des Katheters relativ zu einer im Blutgefäß befindlichen Verengung (Stenose) zu bestimmen. Sobald der Katheter entsprechend positioniert ist, kann der Stent 4 durch Expansion appliziert werden und die Verengung aufweiten.

Ist der Stent 4 leitfähig und in direktem Blutkontakt, so wird der Messstrom hauptsächlich über den Stentkörper fließen und die wesentlichen, zu messenden Impedanzänderungen entstehen an den beiden Übergangsbereichen zwischen den Elektroden 2, 3 und dem Stent 4. So kann die Lage des Katheters relativ zur Verengung, und damit auch die Lage des Stents 4, anhand des Impedanzverlaufs gemäß Figur 2 bestimmt werden. Ist der Abstand zwischen den beiden Elektroden 2 und 3 kürzer als die längsaxiale Ausdehnung der zu behandelnden Verengung, so kommt es zu einem zweistufigen Impedanzverlauf (A). Eine erste stufenförmige Impedanzzunahme zeigt den Eintritt der Elektrode 3 in den Bereich der Verengung an. Eine darauf additiv aufsetzende zweite stufenförmige Impedanzzunahme zeigt dann den Eintritt der Elektrode 2 in den Bereich der Verengung an. Das Impedanzplateau zwischen dem ersten und zweiten stufenförmigen Impedanzanstieg entspricht dem Abstand zwischen den beiden Elektroden 2 und 3. Beim Verlassen des Bereichs der Verengung ergibt sich das komplementäre Bild für die stufenförmigen Impedanzabnahmen. Für den Fall, dass der Elektrodenabstand mit der Länge der Verengung übereinstimmt ergibt sich eine einzige stufenförmige Impedanzzunahme, gefolgt von einem Impedanzplateau dessen Länge dem doppelten Abstand der beiden Elektroden 2 und 3 voneinander entspricht (B). Wenn jedoch der Elektrodenabstand größer ist als die Länge der Verengung, dann ergibt sich nach einem ersten stufenförmigen Impedanzanstieg bei Eintritt der Elektrode 3 in den Bereich der Verengung, gefolgt von einem Impedanzplateau mit der Länge der Verengung. An dieses Impedanzplateau schließt sich dann eine stufenförmige Impedanzabnahme an, die den Austritt der Elektrode 3 aus dem verengten Bereich anzeigt. Die Impedanz steigt dann erst wieder an, wenn die Elektrode 2 in den Bereich der Verengung eintritt. Das Impedanzplateau zwischen der stufenförmigen Impedanzabnahme der Elektrode 3 und der stufenförmigen Impedanzzunahme der Elektrode 2 gibt die Länge an, um die der Abstand zwischen den beiden Elektroden 2 und 3 die längsaxiale Ausdehnung der Verengung übersteigt. Wird der Katheter so positioniert, dass sich die Impedanz zwischen der ersten Impedanzabnahme und der zweiten Impedanzzunahme befindet, so ist davon auszugehen, das sich der Stent 4 im Bereich der Verengung befindet und über den gesamten zu behandelnden Bereich erstreckt. Der Katheter befindet sich also in einer Position in der der Stent 4 durch Expansion appliziert werden kann. Die Ideale Position für die Applikation des Stents 4 liegt dann in der Mitte zwischen dem ersten Impedanzabfall und dem zweiten Anstieg.

Ist der Stent 4 nicht leitfähig oder steht der Stent 4 während des Vorschubs des Katheters nicht in elektrisch leitendem Blutkontakt, dann ändern sich zwar die Muster der Impedanzverläufe, es ist jedoch trotzdem eine eindeutige Positionierung des Katheters sowie eine Überprüfung der Verengungslänge möglich, wie in Figur 3 dargestellt. Ist der Abstand zwischen den beiden Elektroden 2 und 3 kürzer als die zu behandelnde Verengung, so ergibt sich ein rampenförmiger Impedanzanstieg (A) der beginnt, wenn die Elektrode 3 in die Verengung eintritt und endet wenn die Elektrode 2 ebenfalls in den Bereich der Verengung vollständig eingetreten ist. Die Länge dieser Rampe entspricht dem Abstand der beiden Elektroden 2 und 3 voneinander. Ist der Abstand der beiden Elektroden 2 und 3 voneinander mit der Länge der Verengung identisch, dann nimmt der Impedanzverlauf die Form eines Dreiecks an (B). Ist der Abstand zwischen den beiden Elektroden 2 und 3 größer als die Verengung dann bildet sich eine Rampe von gleicher Länge wie die zu behandelnden Verengung aus (C). Daran schließt sich ein Impedanzplateau an, dessen Länge den Unterschied zwischen der Länge der Verengung und dem Abstand zwischen den Elektroden 2 und 3 abbildet. Wird der Katheter so positioniert, dass sich die Impedanz in diesem Impedanzplateau zwischen dem rampenförmigen Impedanzanstieg und der darauffolgenden rampenförmigen Impedanzabnahme befindet, so ist davon auszugehen, das sich der Stent 4 im Bereich der Verengung befindet und über den gesamten, zu behandelnden Bereich erstreckt. Der Katheter befindet sich also in einer Position in der der Stent 4 durch Expansion appliziert werden kann. Die ideale Position für die Applikation des Stents 4 liegt dann in der Mitte dieses Impedanzplateaus.

## Patentansprüche

1. Verfahren zur Bestimmung der Lage eines Katheters in einem Blutgefäß relativ zu einer Veränderung des Gefäßquerschnitts, insbesondere einerVerengung oder Erweiterung des Blutgefäßes, umfassend die Schritte:
- Bereitstellung eines Katheters der mindestens zwei Elektroden aufweist, die während der Positionierung des Katheters im Blutgefäß mit dem umgebenden Blut elektrisch leitend kontaktierbar sind, wobei die beiden Elektroden auf dem Katheter entlang der longitudinalen Achse in einem festgelegten Abstand voneinander angebracht sind;
- Vorschub des Katheters im zu behandelnden Gefäß auf die Veränderung des Querschnitts zu; und
- Messung der Impedanz über die beiden Elektroden während des Vorschubs des Katheters.

2. Verfahren nach Anspruch 1, wobei der gemessene Impedanzverlauf während des Vorschubs für die Bestimmung der Lage des Katheters im Blutgefäß entlang der longitudinalen Achse und relativ zu einer Veränderung des Gefäßquerschnitts verwendet wird.

3. Verfahren nach einem der vorhergehenden Ansprüche, wobei die Veränderung in der Impedanz über die beiden Elektroden kontinuierlich oder diskontinuierlich bestimmt wird.

4. Verfahren nach einem der vorhergehenden Ansprüche, wobei der Impedanzverlauf als Impedanz als Funktion der Strecke des Vorschubs bestimmt wird.

5. Verfahren nach einem der vorhergehenden Ansprüche, wobei der Katheter zwischen den beiden Elektroden einen dilatierbaren Bereich aufweist.

6. Verfahren nach einem der vorhergehenden Ansprüche, wobei der Katheter zwischen den beiden Elektroden einen Stent trägt.

7. Verfahren nach Anspruch 6, wobei der Katheter im Blutgefäß derart positioniert wird, dass der Stent im Bereich der Veränderung des Gefäßquerschnitts applizierbar ist.

8. Verfahren nach Anspruch 6 oder 7, wobei der Katheter einen leitfähigen Stent trägt, der während der Positionierung des Katheters im Blutgefäß mit dem umgebenden Blut elektrisch leitend kontaktierbar ist.

9. Verfahren nach Anspruch 8, wobei Eintritt und Austritt jeder der beiden Elektroden aus dem Bereich der Veränderung des Gefäßquerschnitts jeweils zu einer stufenförmigen Veränderung der Impedanz über die beiden Elektroden führt.

10. Verfahren nach Anspruch 9, wobei der Katheter im Blutgefäß derart positioniert wird, dass sich die Impedanz über die beiden Elektroden zwischen der stufenförmigen Impedanzveränderung der bereits aus dem Bereich der Veränderung des Gefäßquerschnitts austretenden ersten Elektrode und vor der stufenförmigen Impedanzveränderung durch die in den Bereich der Veränderung des Gefäßquerschnitts eintretenden zweiten Elektrode befindet.

11. Verfahren nach Anspruch 5, 6 oder 7, wobei der Katheter keinen Stent trägt, einen im Wesentlichen nicht leitfähigen Stent trägt oder der Stent während der Positionierung des Katheters im Blutgefäß nicht mit dem umgebenden Blut elektrisch leitend kontaktierbar ist.

12. Verfahren nach Anspruch 11, wobei Eintritt und Austritt jeder der beiden Elektroden aus dem Bereich der Veränderung des Gefäßquerschnitts jeweils zu einer rampenförmigen Veränderung der Impedanz über die beiden Elektroden führt.

13. Verfahren nach Anspruch 12, wobei der Katheter im Blutgefäß derart positioniert wird, dass sich der Impedanzverlauf in einem Impedanzplateau befindet, welches sich zwischen einer rampenförmigen Impedanzzunahme und einer rampenförmigen Impedanzabnahme befindet.

14. Verwendung eines Verfahrens nach einem der Ansprüche 1 bis 12 zur Platzierung eines Stents in einem Blutgefäß, zur Bestimmung der Ausdehnung einer Veränderung des Gefäßquerschnitts und/oder zur Kontrolle des Therapieerfolges nach erfolgter Aufweitung und/oder Stabilisierung einer Verengung oder Erweiterung des Blutgefäßes durch Ballondilatation oder Stentapplikation.

15. Katheter zur Verwendung in einem Verfahren nach einem der Ansprüche 1 bis 12.
